# EUROPÄISCHE PATENTSCHRIFT

(11) **EP 0 668 063 B1**
(45) Veröffentlichungstag und Bekanntmachung des Hinweises auf die Patenterteilung: **14.07.1999**
(21) Anmeldenummer: 93810558.2
(22) Anmeldetag: 09.08.1993
(51) Int. Cl.: A61F 2/34

(54) **Schale für eine Gelenkprothese, insbesondere Hüftgelenkprothese und Einsatz zur Verwendung mit der Schale**
Shell for joint prosthesis, especially hip joint prosthesis and insert for use with the shell
Coupe pour prothèse d'articulation, notamment prothèse de la hanche et insertion pour l'utilisation avec la coupe

(43) Veröffentlichungstag der Anmeldung: 23.08.1995
(73) Patentinhaber: Tschirren, Ernst jun., 3126 Gelterfingen (CH)
(72) Erfinder: Tschirren, Ernst, CH-3074 Muri/BE (CH)
(74) Vertreter: Tschudi, Lorenz

(56) Entgegenhaltungen:
- EP-A- 0 483 023
- EP-A- 0 552 949
- FR-A- 2 416 004
- FR-A- 2 649 005
- FR-A- 2 653 659
- GB-A- 1 126 961
- US-A- 4 936 863

## Beschreibung

Die vorliegende Erfindung bezieht sich auf eine Schale für eine Gelenkprothese, insbesondere eine Hüftgelenkprothese gemäss dem Oberbegriff des Patentanspruches 1 und eine Gelenkprothese gemäss dem Oberbegriff des Patentanspruches 5.

Bisher wurden bei Gelenkprothesen, insbesondere Hüftgelenkprothesen, Schalen aus Polyethylen in den Hüftknochen einzementiert. Im Laufe der Jahre, wenn Beschädigungen auftraten, musste die Schale entfernt werden, wobei es beim Wechsel zum Verlust von Knochensubstanz kam. Ein weiterer Nachteil war der grosse Polyethylenabrieb. Als andere Möglichkeit wurden Schraubringe in das Hüftgelenk zum Einsetzen der Pfanne eingeschraubt, die sich jedoch mit der Zeit lockerten. Die Verwendung von Schraubringen aus Keramik hatte den Vorteil, dass kein Polyethylenabrieb auftrat.

Aus der FR-A-2649005 ist eine Schale für eine Hüftgelenkprothese vorbekannt, die eine sich vom Innenbereich an den Rand der Schale erstreckende Öffnung mit Seitenrändern aufweist, wobei der Rand der Schale mit einem umlaufenden ersten Organ zum Eingriff mit einem Einsatz versehen ist. Die Schale gemäss der FR-A-2649005 weist den Nachteil auf, dass keine Einbuchtungen zum Ansetzen einer Zange vorgesehen sind. Aus der EP-0483023 ist ein Implantat vorbekannt, dessen Schale eine variable Dicke aufweist. Diese Schale bildet den Oberbegriff des Anspruchs 1. Der Gegenstand gemäss der GB-1126961 ermöglicht nicht ein Anbringen des Ansatzes in der Schale und der Nut im Einsatz.

Es ist eine Aufgabe der vorliegenden Erfindung, eine Schale für eine Gelenkprothese, insbesondere eine Hüftgelenkprothese und eine Gelenkprothese zu schaffen, welche die Nachteile des Standes der Technik nicht aufweisen. Die Schale soll mit einem Instrument etwas deformiert werden können, so dass der Einsatz beim Wechsel der Gelenkprothese herausgehoben werden kann und nicht herausgemeisselt werden muss. Im weiteren soll eine optimale Krafteinleitung vom mit der Kugel belasteten Einsatz in die Schale stattfinden. Zusätzlich soll ein gewisser Federungseffekt auftreten, der die Mikrobewegungen des Beckens und Schläge auffängt und den Keramikeinsatz schont. Dies wird erzielt durch die Merkmale der Ansprüche 1 und 5. Bevorzugte Ausführungsformen ergeben sich aus den abhängigen Ansprüchen.

Im folgenden wird anhand der beiliegenden Zeichnung ein Ausführungsbeispiel der Erfindung sowie dessen Verwendung näher beschrieben. Es zeigen
- **Fig. 1**: einen teilweisen Schnitt durch die Hüftgelenkprothese mit Schale, Einsatz und Kugel,
- **Fig. 2**: eine Draufsicht auf die Schale der Hüftgelenkprothese,
- **Fig. 3**: einen Schnitt durch die Schale gemäss Linie III-III der Fig. 2,
- **Fig. 4**: eine Ansicht der Schale gemäss Pfeil A der Fig. 2,
- **Fig. 5**: ein Detail der Prothese bei zusammengefügter Schale und Einsatz,
- **Fig. 6**: ein Detail der Prothese, ähnlich wie in der Fig. 5.

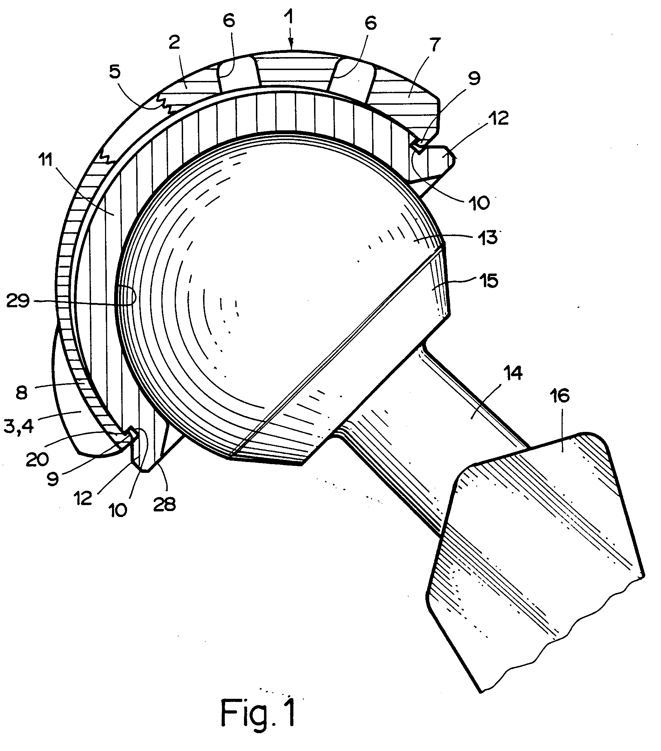

Die in Fig. 1 dargestellte Hüftgelenkprothese 1 wird dann verwendet, wenn die Pfanne des Hüftknochens beschädigt ist. Die als teilkugeliges Hohlelement ausgebildete Metallschale 2 aus einer Titan/Aluminiumlegierung, vorzugsweise TA₆V₄, wird in die in Fig. 1 nicht dargestellte Pfanne des Hüftknochens eingesetzt, wobei die Flügel 3, 4 in den Knochen hineingedrückt werden. Die Schale ist selbstspannend im Hüftknochen. Ein mit einem Aussengewinde versehenes, in der Figur nicht dargestelltes Instrument wird in eine, mit einem Innengewinde versehene Bohrung 5 der Schale eingeschraubt, wobei mit dem Instrument die Schale in die Pfanne des Hüftknochens hineingedrückt wird. Die Schale 2 ist im weiteren mit Durchgangslöchern 6 versehen, durch welche, falls notwendig, Schrauben in den Hüftknochen eingeschraubt werden können. Die Schale ist auf der einen Seite 7 mit einer grösseren Dicke ausgebildet als auf der gegenüberliegenden Seite 8. Dadurch wird eine Federwirkung und eine bessere Krafteinleitung erzielt. Die Schale ist an ihrem Rand mit einem nach innen abstehenden, ringförmig verlaufenden Ansatz 9 versehen, der in eine entsprechende Nut 10 im Einsatz 11 eingreift. Die Nut könnte auch an der Schale und der Ansatz am Einsatz ausgebildet sein. Der Einsatz ist aus Aluminiumoxidkeramik gefertigt. Der Einsatz, der etwa die Form einer halben Hohlkugel aufweist, ist an seinem Rand mit einem ringförmigen Absatz 12, der eine Randfläche 28 aufweist, versehen. Die im Einsatz 11 gelagerte Gelenkkugel 13 ist ebenfalls aus Aluminiumoxidkeramik gefertigt. Die Kugel 13 ist im Bereich, wo der Schafthals 14 in dieselbe eingeführt ist, mit einer Anschrägung 15 versehen. Der Schafthals 14 ist mit dem Femurschaft 16 verbunden. Der Schafthals und der Femurschaft sind vorzugsweise ebenfalls aus der obgenannten Titanlegierung gefertigt. Der Einsatz ist mindestens an der Innenfläche 29 geschliffen.

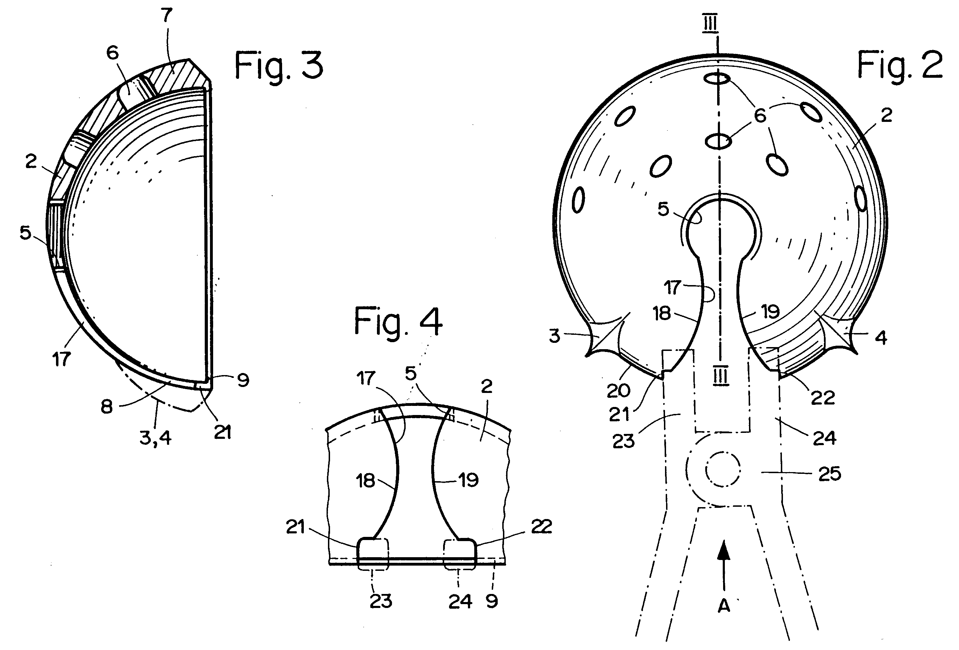

Fig. 2 zeigt eine Draufsicht auf die Schale 2. Anschliessend an die mit einem Innengewinde versehene etwa kreisförmige mittlere Oeffnung 5 schliesst sich eine schlitzförmige Oeffnung 17 an, die sich bis an den Rand 20 der Schale 2 erstreckt. Die Ränder 18 und 19 der Oeffnung 17 sind abgerundet ausgebildet. An ihrem dem Rand 20 der Schale benachbarten Enden sind die Ränder 18 und 19 der Oeffnung 17 mit Einbuchtungen 21 und 22 versehen. Mit den Enden 23 und 24 einer Zange 25 kann die Schale 2 auseinandergedrückt werden, um den Einsatz 11 aus der Schale herauszulösen, falls ein neues Gelenkimplantat eingesetzt werden muss. Es muss nicht, wie bisher das alte Implantat, teilweise mit dem Meissel entfernt werden.

Fig. 3 zeigt einen Schnitt gemäss Linie III-III der Fig. 2.

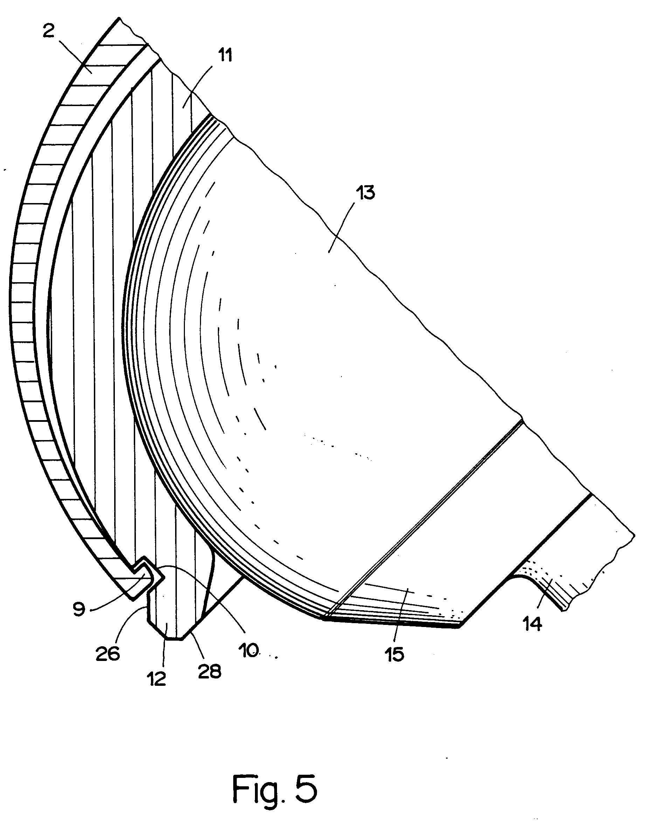

Fig. 4 zeigt eine Ansicht gemäss Pfeil A der Fig. 2.

In Fig. 5 ist der Bereich des Eingriffs der Schale 2 mit dem Einsatz 11 vergrössert dargestellt. Die Kraftübertragung erfolgt vom Femurschaft 16 über den Schafthals 14 auf die Kugel 13, von dieser auf den Einsatz 11 und vom Ende 12 des Einsatzes auf den Ansatz 9 der Schale 2. Die Schale 2 berührt im übrigen Bereich den Einsatz nicht, so dass die Krafteinleitung am Rand des Einsatzes von diesem auf die Schale optimal erfolgt. Die Krümmungsradien der Schale 2 und des Einsatzes 11 sind so gewählt, dass Schale und Einsatz nur an ihren Randbereichen Kontakt aufweisen. Somit nimmt die Schale 2 die vom Bein auf den Femurschaft ausgeübten Kräfte optimal auf und verteilt sie auf die Gelenkpfanne im Hüftknochen. Die Kugel und der Einsatz weichen von der Kugelgestalt etwas ab, damit sich die Gelenkflüssigkeit gut verteilt.

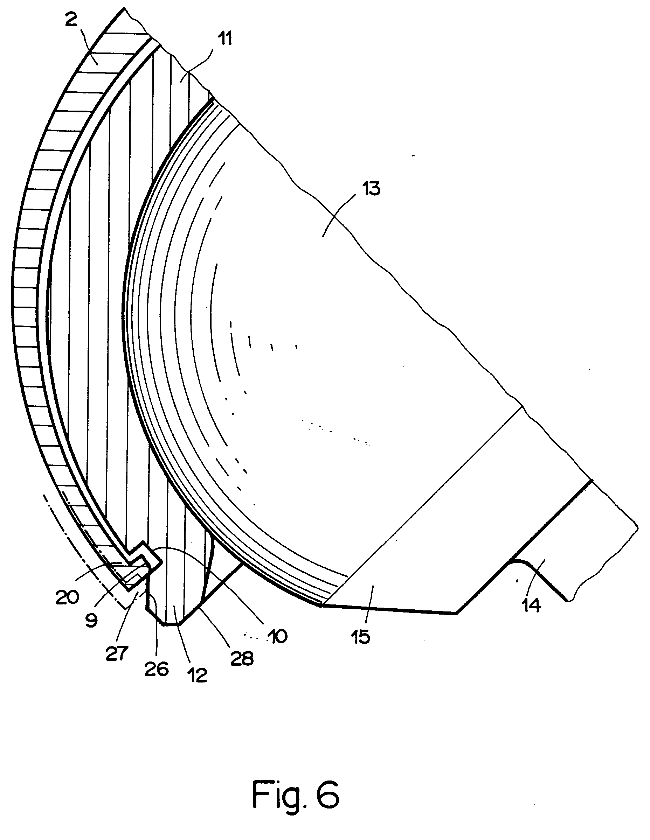

Fig. 6 zeigt ebenfalls in vergrösserter Darstellung den Bereich, wo die Schale 2 in Eingriff mit dem Einsatz 11 steht. Werden durch grösse Krafteinwirkung die Schale 2 und der Einsatz 11 gegeneinander gedrückt, so kann die Schale mit dem Ansatz 9 an ihrem Ende über eine Fläche 26 am Absatz 12 nach unten in die strichpunktiert gezeigte Position 27 gelangen, ohne dass der Einsatz beschädigt wird, d.h. insbesondere, ohne dass der Absatz abbricht. Die Fläche 26 ist gegenüber der Randfläche 28 vorzugsweise um etwa 45° geneigt.

## Patentansprüche

1. Schale (2), insbesondere für eine Hüftgelenkprothese, wobei sie eine sich von einer mittleren Öffnung (5) an den Rand (20) der Schale (2) erstreckende Öffnung (17) mit Seitenrändern (18, 19) aufweist, wobei die Schale (2) auf der einen Seite (7) eine grössere Dicke aufweist als auf der gegenüberliegenden Seite (8), dadurch gekennzeichnet, dass die Seitenränder je mit mindestens einer Einbuchtung (21, 22) zum Ansetzen einer Zange versehen sind, und der Rand (20) der Schale mit einem umlaufenden Ansatz (9) oder einer Nut zum Eingriff mit einem Einsatz (11) versehen ist, wobei die Einbuchtungen (21, 22) am Übergang der Seitenränder (18, 19) der Öffnung (17) zum Rand (20) der Schale (2) angeordnet sind.

2. Schale nach Patentanspruch 1, dadurch gekennzeichnet, dass sie als teilkugeliges Hohlelement ausgebildet ist.

3. Schale nach einem der vorangehenden Patentansprüche, dadurch gekennzeichnet, dass die mittlere Öffnung (5) ein Innengewinde aufweist.

4. Schale nach einem der vorangehenden Patentansprüche, dadurch gekennzeichnet, dass sie aus einer Titan/Aluminiumlegierung besteht.

5. Gelenkprothese mit einer Schale (2) nach einem der Patentansprüche 1 bis 4 und einem mit der Schale lösbar verbundenen Einsatz (11), dadurch gekennzeichnet, dass der Ansatz (9) der Schale (2) in Eingriff mit einer Nut (10) des Einsatzes (11) steht, wobei sich die Schale (2) und der Einsatz (11) nur im Bereich des Ansatzes (9) und der Nut (10) berühren, wodurch eine optimale Krafteinleitung von der Gelenkkugel (13) über den Einsatz (11) in die Schale (2) gewährleistet wird.

6. Gelenkprothese nach Patentanspruch 5, dadurch gekennzeichnet, dass der Einsatz (11) benachbart seiner Randfläche (28) mit der Nut (10) versehen ist und der Einsatz zwischen Nut (10) und Randfläche (28) einen mit einer abgeschrägten Fläche (26) versehenen Absatz (12) aufweist, so dass bei Krafteinwirkung die Schale (2) mit dem Ansatz (9) an ihrem Ende über die abgeschrägte Fläche (26) in eine untere Position gelangen kann, ohne dass der Einsatz beschädigt wird.

7. Gelenkprothese nach Patentanspruch 6, dadurch gekennzeichnet, dass die abgeschrägte Fläche (26) etwa einen Winkel von 45° mit der Randfläche (28) einschliesst.

8. Gelenkprothese nach einem der Patentansprüche 5, 6 oder 7, dadurch gekennzeichnet, dass der Einsatz etwa die Form einer halben Hohlkugel aufweist.

## Claims

1. Cup (2), in particular for a hip joint prosthesis, it having an aperture (17) with side edges (18, 19), said aperture extending from a central opening (5) to the rim (20) of the cup (2), the cup (2) having a greater thickness on the one side (7) than on the opposite side (8), characterised in that the side edges are each provided with at least one niche (21, 22) for placement of pincers, and the rim (20) of the cup is provided with an encircling flange (9) or a groove for engagement with an insert (11), the niches (21, 22) being disposed at the transition of the side edges (18, 19) of the aperture (17) to the rim (20) of the cup (2).

2. Cup according to claim 1, characterised in that it is designed as a partially spherical, hollow element.

3. Cup according to one of the preceding claims, characterised in that the central opening (5) has an inner threading.

4. Cup according to one of the preceding claims, characterised in that it consists of a titanium/aluminum alloy.

5. Joint prosthesis with a cup (2) according to one of the claims 1 to 4 and an insert (11) detachably connected to the cup, characterised in that the flange (9) of the cup (2) is in engagement with a groove (10) of the insert (11), the cup (2) and the insert (11) only touching in the region of the flange (9) and the groove (10), whereby an optimal introduction of force from the joint ball head (13) via the insert (11) into the cup (2) is ensured.

6. Joint prosthesis according to claim 5, characterised in that the insert (11) is provided with the groove (10) adjacent to its edge face (28), and between the groove (10) and the edge face (28) the insert has a shoulder (12) with a chamfered surface (26) so that during the influence of force the cup (2) with the flange (9) can reach a lower position at its end via the chamfered surface (26) without the insert being damaged.

7. Joint prosthesis according to claim 6, characterised in that the chamfered surface (26) encloses approximately an angle of 45° with the edge face (28).

8. Joint prosthesis according to one of the claims 5, 6, or 7, characterised in that the insert has approximately the shape of a half of a hollow sphere.

## Revendications

1. Coupe (2), en particulier destinée à une prothèse de hanche, présentant un orifice (17) s'étendant à partir d'un orifice central (5) sur le bord (20) de la coupe (2) comportant des bords latéraux (18, 19), la coupe (2) présentant sur un côté (7) une épaisseur plus importante que sur le côté opposé (8), caractérisée en ce que les bords latéraux sont respectivement pourvus au minimum d'une endenture (21, 22) pour l'application d'une pince et que le bord (20) de la coupe est doté d'un épaulement (9) périphérique ou d'une rainure pour s'engager dans une insertion (11), les endentures (21, 22) étant disposées sur la transition des bords latéraux (18, 19) de l'orifice (17) par rapport au bord (20) de la coupe (2).

2. Coupe conforme à la revendication 1, caractérisée en ce qu'elle est constituée par un élément creux partiellement sphérique.

3. Coupe conforme à l'une des revendications précédentes du brevet, caractérisée en ce que l'orifice central (5) présente un filetage intérieur.

4. Coupe conforme à l'une des revendications précédentes du brevet, caractérisée en ce qu'elle est constituée d'un alliage d'aluminium/titane.

5. Prothèse de hanche comportant une coupe (2) conforme à l'une des revendications 1 à 4 du brevet et comportant une insertion (11) reliée de façon désolidarisable à la coupe, caractérisée en ce que l'épaulement (9) de la coupe (2) s'engage dans une rainure (10) de l'insertion (11), la coupe (2) et l'insertion (11) n'étant en contact que dans le domaine de l'épaulement (9) et de la rainure (10), ce qui garantit une application optimale de la force de la rotule de l'articulation (13) dans la coupe (2) par l'intermédiaire de l'insertion (11).

6. Prothèse de hanche conforme à la revendication 5 du brevet, caractérisée en ce que l'insertion (11) est pourvue, à côté de la surface du bord (28) de la rainure (10) et que l'insertion présente entre la rainure (10) et la surface du bord (28) un talon (12) pourvu d'une surface chanfreinée (26), de telle manière que, lors de l'application de la force, la coupe (2) puisse parvenir dans une position inférieure sur son extrémité par l'intermédiaire de la surface chanfreinée (26) sans que l'insertion soit endommagée.

7. Prothèse de hanche conforme à la revendication 6 du brevet, caractérisée en ce que la surface chanfreinée (26) constitue un angle d'environ 45° avec la surface du bord (28).

8. Prothèse de hanche conforme à l'une des revendications 5 ou 6 du brevet, caractérisée en ce que l'insertion présente approximativement la même forme qu'une demi-sphère creuse.
